# EUROPÄISCHE PATENTSCHRIFT

(11) **EP 2 214 733 B1**
(45) Veröffentlichungstag und Bekanntmachung des Hinweises auf die Patenterteilung: **12.10.2016**
(21) Anmeldenummer: 08842842.0
(22) Anmeldetag: 17.10.2008
(51) Int. Cl.: A61L 27/24, A61L 27/50, A61L 27/54

(54) **BIOVERBUNDMATERIAL FÜR DIE KONTROLLIERTE FREISETZUNG VON WIRKSTOFFEN**
COMPOSITE BIOMATERIAL FOR CONTROLLED RELEASE OF ACTIVE INGREDIENTS
MATÉRIAU COMPOSITE BIOLOGIQUE POUR LA LIBÉRATION CONTRÔLÉE D'AGENTS ACTIFS

(30) Priorität: 18.10.2007 DE 102007051059
(43) Veröffentlichungstag der Anmeldung: 11.08.2010
(73) Patentinhaber: NMI NATURWISSENSCHAFTLICHES UND MEDIZINISCHES INSTITUT AN DER UNIVERSITÄT TÜBINGEN, 72770 Reutlingen (DE)
(72) Erfinder: STOLL, Dieter, 72116 Moessingen (DE); STOOP, Reinout, NL-2251 PJ Voorschoten (NL); VAZ NUERNBERGER, Juliana, 70180 Stuttgart (DE); GAISSMAIER, Christoph, 72127 Kusterdingen-Mähringen (DE); AHLERS, Michael, 69412 Eberbach (DE); BOHNSACK, Claudia, 22043 Hamburg (DE)
(74) Vertreter: Witte, Weller & Partner Patentanwälte mbB
(86) Internationale Anmeldenummer: PCT/EP2008/008801
(87) Internationale Veröffentlichungsnummer: WO 2009/052998

(56) Entgegenhaltungen:
- EP-A2- 1 908 484
- WO-A1-97/41899
- WO-A1-2009/006558
- WO-A2-2007/043048
- WO-A2-2007/057175

### SEQUENZPROTOKOLL

<110> NM Naturwissenschaftliches und Medizinisches Institut an der Uni ver si t ät Tübingen
<120> Bi over bundmat er i al für die kontrollierte Freisetzung von Wirkstoffen
<130> 3605P140WO
<150> DE 10 2007 051 059.6
   <151 > 2007-10-18
<160> 29
<170> Pat ent In Version 3.3
<210> 1
   <211> 19
   <212> PRT
   <213> Künstliche Sequenz
<220>
   <223> Kei ne
<400> 1
   Cys Asp Ile His Val Leu Lys Gln Asp Gly Gln Phe Ser Val Leu Phe
   1               5                   10                  15
   Thr Lys Cys
<210> 2
   <211> 19
   <212> PRT
   <213> Künstliche Sequenz
<220>
   <223> Keine
<400> 2
   Cys Asp Ile His Val Leu Lys Gln Asp Gly Gln Phe Ser Val Leu Phe
   1 5 10 15
   Thr Lys Cys
<210> 3
   <211> 19
   <212> PRT
   <213> Künstliche Sequenz
<220>
   <223> Keine
<400> 3
   Lys Asp Ile His Val Leu Lys Gln Asp Gly Gln Phe Ser Val Leu Phe
   1 5 10 15
   Thr Lys Gly
<210> 4
   <211> 19
   <212> PRT
   <213> Künstliche Sequenz
<220>
   <223> Keine
<400> 4
   Lys Asp Ile His Val Leu Lys Gln Asp Gly Gln Phe Ser Val Leu Phe
   1 5 10 15
   Thr Lys Gly
<210> 5
   <211> 19
   <212> PRT
   <213> Künstliche Sequenz
<220>
   <223> Keine
<400> 5
   Cys Arg Ser Thr Val Arg Met Ser Ala Glu Gln Val Gln Asn Val Trp
   1 5 10 15
   Val Arg Cys
<210> 6
   <211> 19
   <212> PRT
   <213> Künstliche Sequenz
<220>
   <223> Keine
<400> 6
   Cys Arg Ser Thr Val Arg Met Ser Ala Glu Gln Val Gln Asn Val Trp
   1 5 10 15
   Val Arg Cys
<210> 7
   <211> 19
   <212> PRT
   <213> Künstliche Sequenz
<220>
   <223> Kei ne
<400> 7
   Lys Arg Ser Thr Val Arg Met Ser Ala Glu Gln Val Gln Asn Val Trp
   1 5 10 15
   Val Arg Gly
<210> 8
   <211> 19
   <212> PRT
   <213> Künstliche Sequenz
<220>
   <223> Keine
<400> 8
   Lys Arg Ser Thr Val Arg Met Ser Ala Glu Gln Val Gln Asn Val Trp
   1 5 10 15
   Val Arg Gly
<210> 9
   <211> 18
   <212> PRT
   <213> Künstliche Sequenz
<220>
   <223> Keine
<400> 9
   Cys Val Ala Val Trp Arg Lys Asn Asp Glu Asn Ile Thr Leu Glu Thr
   1               5                   10                  15
   Val Cys
<210> 10
   <211> 18
   <212> PRT
   <213> Künstliche Sequenz
<220>
   <223> Keine
<400> 10
   Cys Val Ala Val Trp Arg Lys Asn Asp Glu Asn Ile Thr Leu Glu Thr
   1               5                   10                  15
   Val Cys
<210> 11
   <211> 18
   <212> PRT
   <213> Künstliche Sequenz
<220>
   <223> Keine
<400> 11
   Lys Val Ala Val Trp Arg Lys Asn Asp Glu Asn Ile Thr Leu Glu Thr
   1               5                   10                  15
   Val Gly
<210> 12
   <211> 18
   <212> PRT
   <213> Künstliche Sequenz
<220>
   <223> Keine
<400> 12
   Lys Val Ala Val Trp Arg Lys Asn Asp Glu Asn Ile Thr Leu Glu Thr
   1               5                   10                  15
   Val Gly
<210> 13
   <211> 8
   <212> PRT
   <213> Künstliche Sequenz
<220>
   <223> Keine
<400> 13
   Tyr Pro Ile Ala Phe Thr Ile Pro
   1 5
<210> 14
   <211> 8
   <212> PRT
   <213> Künstliche Sequenz
<220>
   <223> Keine
<400> 14
   Val Pro Asn Glu Tyr Gln Lys Thr
   1 5
<210> 15
   <211> 8
   <212> PRT
   <213> Künstliche Sequenz
<220>
   <223> Keine
<400> 15
   Ala His Asn Lys Val Gln Phe Tyr
   1 5
<210> 16
   <211> 8
   <212> PRT
   <213> Künstliche Sequenz
<220>
   <223> Keine
<400> 16
   Gly Leu Ala Gly Gln Arg Gly Ile
   1 5
<210> 17
   <211> 8
   <212> PRT
   <213> Künstliche Sequenz
<220>
   <223> Keine
<400> 17
   Arg Thr Pro Phe Gln Ala Asp Ser
   1 5
<210> 18
   <211> 8
   <212> PRT
   <213> Künstliche Sequenz
<220>
   <223> Keine
<400> 18
   Asp Gln Thr Tyr Gly Pro Thr His
   1 5
<210> 19
   <211> 8
   <212> PRT
   <213> Künstliche Sequenz
<220>
   <223> Keine
<400> 19
   Gly Gln Pro Val Lys Gly Arg Glu
   1 5
<210> 20
   <211> 8
   <212> PRT
   <213> Künstliche Sequenz
<220>
   <223> Keine
<400> 20
   Thr Trp Val Tyr His Gly Val Asp
   1 5
<210> 21
   <211> 8
   <212> PRT
   <213> Künstliche Sequenz
<220>
   <223> Keine
<400> 21
   Gly Pro Leu Gly Val Arg Gly Lys
   1 5
<210> 22
   <211> 8
   <212> PRT
   <213> Künstliche Sequenz
<220>
   <221> misc_feat ur e
   <222> (1)..(1)
   <223> Xaa kann jede natürlich vorkommende Aminosäure sei n
<400> 22
   Xaa Pro Leu Gly Val Arg Gly Lys
   1               5
<210> 23
   <211> 8
   <212> PRT
   <213> Künstliche Sequenz
<220>
   <221> misc_feat ur e
   <222> (2).. (2)
   <223> Xaa kann jede natürlich vorkommende Aminosäure sein
<400> 23
   Gly Xaa Leu Gly Val Arg Gly Lys
   1               5
<210> 24
   <211> 8
   <212> PRT
   <213> Künstliche Sequenz
<220>
   <221> misc_f eat ur e
   <222> (3).. (3)
   <223> Xaa kann jede natürlich vorkommende Aminosäure sein
<400> 24
   Gly Pro Xaa Gly Val Arg Gly Lys
   1               5
<210> 25
   <211> 8
   <212> PRT
   <213> Künstliche Sequenz
<220>
   <221> misc_f eature
   <222> (4).. (4)
   <223> Xaa kann jede natürlich vorkommende Aminosäure sei n
<400> 25
   Gly Pro Leu Xaa Val Arg Gly Lys
   1 5
<210> 26
   <211> 8
   <212> PRT
   <213> Künstliche Sequenz
<220>
   <221> misc_feature
   <222> (5).. (5)
   <223> Xaa kann jede natürlich vorkommende Aminosäur sein
<400> 26
   Gly Pro Leu Gly Xaa Arg Gly Lys
   1 5
<210> 27
   <211> 8
   <212> PRT
   <213> Künstliche Sequenz
<220>
   <221> misc_f eature
   <222> (6).. (6)
   <223> Xaa kann jede natürlich vorkommende Aminosäure sein
<400> 27
   Gly Pro Leu Gly Val Xaa Gly Lys
   1 5
<210> 28
   <211> 8
   <212> PRT
   <213> Künstliche Sequenz
<220>
   <221> misc_feature
   <222> (7)..(7)
   <223> Xaa kann jede natürlich vorkommende Aminosäure sein
<400> 28
   Gly Pro Leu Gly Val Arg Xaa Lys
   1 5
<210> 29
   <211> 8
   <212> PRT
   <213> Künstliche Sequenz
<220>
   <221> misc_f eature
   <222> (8).. (8)
   <223> Xaa kann jede natürlich vorkommende Aminosäure sein
<400> 29
   Gly Pro Leu Gly Val Arg Gly Xaa
   1 5

## Beschreibung

Die vorliegende Erfindung betrifft ein Bioverbundmaterial mit kontrollierter Freisetzung von Wirkstoffen, umfassend eine dreidimensionale Trägerstruktur auf Polymerbasis, sowie eine Matrixstruktur auf Polymerbasis.

Bioverbundmaterialien werden im Stand der Technik für verschiedene Zwecke benötigt und eingesetzt, wie beispielsweise im Bereich des sog. Tissue-Engineerings in der regenerativen Medizin. Diese Technik hat zum Ziel, menschliches oder tierisches Gewebe, das in irgendeiner Weise verletzt, geschädigt, degeneriert, o.ä. ist, durch resorbierbare Implantate wieder aufzubauen und funktionsfähig zu machen. Mit den Implantaten, die meist sowohl eine schützende Matrix für das sich neu bildende Gewebe bilden, als auch das Zellwachstums aktiv fördern, soll die Regeneration von betroffenem Gewebe erreicht werden. Für diese Neu-Züchtung von Gewebe werden Zellen eines Organismus entweder außerhalb eines zu regenerierenden Zielgewebes vermehrt und nach der Kultivierung in den gleichen oder einen anderen Organismus zur Gewebeneubildung bzw. -regeneration implantiert. Ferner besteht die Möglichkeit, Wirkstoffe in das Biomaterial einzubinden und nach Implantation dieses Biomaterials in das betroffene Gewebe in das das Biomaterial umgebende Gewebe freizusetzen, wodurch Zellen, die das Biomaterial umgeben und/oder in dieses Einwandern durch die Wirkstoffe bspw. zum Zellwachstum beeinflusst werden können.

Solche Implantate bzw. Biomaterialien sind meist aus mehreren Komponenten hergestellt und bilden daher Verbundmaterialien. Sie werden bspw. bei der GelenkKnorpel-Regeneration eingesetzt. Solche Biomaterialien müssen daher neben bioverträglichen und bio-wirksamen Eigenschaften auch eine hinreichende mechanische Festigkeit und Stabilität aufweisen, um die in Ihnen enthaltenen und/oder aufzunehmenden Zellen zu schützen und um gegenüber *in-vivo* Kräften, die während einer Bewegung auftreten, zu bestehen.

Andererseits finden solche Trägerstrukturen auch ihre Verwendung als Wirkstoff-Freisetzende Biomaterialien ("Drug-Release-Materials"), über welche in die Trägerstruktur eingebundene Wirkstoffe gezielt an der Stelle freigesetzt werden können, an welcher die Trägerstruktur implantiert wird.

Ein wichtiger Aspekt ist dabei für beide Einsätze der Biomaterialien, dass sie biologisch abbaubar und nicht toxisch sind, sowie keine stimulierenden Effekte auf an Entzündungen beteiligte Zellen ausüben. Ferner spielt auch die praktische Handhabbarkeit und Fragen der Zulassung solcher Implantate eine zunehmend größere Rolle.

Eine wesentliche Verbesserung der bisher für diese Einsatzbereiche verwendeten Biomaterialien ist daher auch, dass diese nicht nur als Wirkstoff-Lieferant fungieren, sondern auch auf Zell-Signale bzw. Signalstoffe reagieren, die von den sie umgebenden Zellen/Zellverbänden abgegeben werden. Ferner ist es wünschenswert, dass die Biomaterialien durch auf bzw. in ihnen enthaltene Substanzen oder aufgrund ihrer Struktur oder Zusammensetzung, die Differenzierung, das Wachstum und/oder den Stoffwechsel von Zellen beeinflussen oder sogar steuern können, bspw. durch die Freisetzung von entsprechenden Substanzen aus dem Biomaterial, wie bspw. Wachstumsfaktoren. Gleichzeitig soll aber diese durch das Biomaterial ausgeübte Funktion mit den einzusetzenden Biomaterial-Komponenten kontrollierbar sein. Dies bedeutet, dass die freigesetzten Substanzen im besten Fall den *in-vivo*-Freisetzungs-Profilen der Faktoren entsprechen, die bei der natürlichen Gewebe-Morphogenese oder bei der Gewebe-Neubildung produziert werden.

Ferner ist bei Biomaterialien, die mit freizusetzenden Wirkstoffen beladen sind, oftmals wünschenswert, dass diese kontrolliert freigesetzt werden können, d.h. bspw. mit einer bestimmten Rate und mit einer bestimmten Geschwindigkeit, oder dass sogar zwei oder mehrere Wirkstoffe gezielt mit gleicher oder unterschiedlicher Rate freigesetzt werden können.

Gegenwärtig werden sowohl natürliche als auch synthetische Biomaterialien für die weiter oben erläuterten Anwendungen im Tissue-Engineering und als Drug-Release-Implantate eingesetzt, und dies in den verschiedensten Formen, einschließlich fasriger Strukturen, poröser Schwämme, gewebten Netzstrukturen und Hydrogele.

Im Stand der Technik ist es bekannt, Wachstumsfaktoren direkt an die Biomaterial-Trägerstrukturen zu binden. So zeigten bspw. Young et al. (J. Control Release 109 (2005) 256-274) die direkte Bindung des basischen Fibroblasten-Wachstumsfaktor (bFGF) an Gelatine.

Ein Bioverbundmaterial bestehend aus einer vernetzten Gelatinematrix und einer kollagenen Trägerschicht ist beschrieben in WO 2007/057175

Die WO 2007/043048 offenbart ein Hydrogel umfassend ein faseriges Netzwerk aus einer Vielzahl von kurzen Peptiden, wobei das Hydrogel auch therapeutisch aktive Substanzen enthalten kann.

Die EP 1908 484 A offenbart einen medizinischen Artikel mit einer Struktur aus einem ersten und einem zweiten biodegradierbaren Material. Der Artikel kann zur kontrollierten Freisetzung eines therapeutischen Wirkstoffs dienen.

Schließlich offenbart die WO 2009/006558 A ein Verbundmaterial mit einer ersten und einer zweiten Schicht, wobei die zweite Schicht die erste zumindest teilsweise umgibt. Eine oder beide Schichten können ein Polymer wie bspw. Polycaprolakton und einen Wirkstoff enthalten.

Ferner ist die Verwendung von Hydrogelen oder von Microsphären bekannt. Hydrogele basieren auf hydrophilen Polymeren, die, um eine Auflösung in Wasser zu vermeiden, vernetzt sind. Da Hydrogele große Mengen an Wasser enthalten können, können sie zur Freisetzung von Protein-Arzneistoffen eingesetzt werden. Eine Varian-te der Hydrogele ist die Bildung von sog. Mikrosphären, die mit bspw. Wachstumsfaktoren beladen, und die anschließend in andere Biomaterialien eingebaut werden.

Lutolf et al. (Proc. Natl. Acad. Sci. U.S.A. (2003) 100:5413-5418) generierten ein Hydrogel, wobei in eine PEG(Polyethylenglykol)-tetravinylsulfon enthaltende Lösung ein Integrin-bindendes Peptid, sowie ein Peptid mit einer Matrixmetalloproteasesensitiven Sequenz zugegeben wurde. Ferner wurde Bone morphogenetic Protein 2 (BMP-2) hinzu gegeben, das in nachfolgenden Versuchen durch den Abbau der Hydrogele durch MMPs freigesetzt wurde.

Darüber hinaus sind Gelatinegele mit adhäsiv gebundenen Wachstumsfaktoren als Wundauflage aus der WO 97/41899 A1 bekannt, sowie die Verwendung von superadsorbierenden porösen Hydrogelverbundstoffen aus der EP 0 988 108 B1.

Trotz der im Stand der Technik bekannten und beschriebenen zahlreichen Biomaterialien für einen Einsatz im Tissue-Engineering oder als Drug-Release-Implantate besteht weiterhin ein enormer Bedarf an verbesserten und flexibel einsetzbaren Biomaterialien.

Aufgabe der vorliegenden Erfindung ist es daher, ein Bioverbundmaterial bereitzustellen, mit welchen eine zeitlich und/oder mengenmäßig kontrollierte Freisetzung von einem oder mehreren Wirkstoffen aus dem Biomaterial ermöglicht wird, wobei gleichzeitig die Herstellung des Biomaterials einfach ist, eine gewisse Flexibilität und Vorteile bei der Herstellung und Zulassung bietet.

Diese Aufgabe wird vorliegend durch ein Bioverbundmaterial gelöst, umfassend eine dreidimensionale Trägerstruktur auf Polymerbasis, sowie eine Matrixstruktur auf Polymerbasis, wobei die Wirkstoffe über die Matrixstruktur in das Bioverbundmaterial eingelagert sind, und wobei das Bioverbundmaterial durch Einbringen der die Wirkstoffe aufweisende Matrixstruktur in die Trägerstruktur erhältlich ist.

Die Aufgabe wird ferner durch ein Verfahren zur Herstellung eines Bioverbundmaterials, umfassend eine dreidimensionale Trägerstruktur auf Polymerbasis, sowie eine Matrixstruktur auf Polymerbasis, gelöst, das die folgenden Schritte aufweist:
a) Bereitstellen von i) Trägermaterial auf Polymerbasis und ii) Matrixmaterial auf Polymerbasis, ggf. mit einem gebundenem Wirkstoff;
b) ggf. Hydrophilisieren des Trägermaterials; und
c) Einbringen des wirkstoffdotierten Matrixmaterials in das hydrophilisierte Trägermaterial aus Schritt b) zur Herstellung eines Bioverbundmaterials mit einer Trägerstruktur und einer Matrixstruktur, die ggf. Wirkstoffdotiert ist.

Die der Erfindung zugrunde liegende Aufgabe wird auf diese Weise vollkommen gelöst.

Mit dem erfindungsgemäßen Bioverbundmaterial wird eine Matrix bereitgestellt, die ein flexibles Ausgangsmaterial für zahlreiche medizinische, biologische und labortechnische Anwendungen ist. Das erfindungsgemäße Bioverbundmaterial bietet gegenüber im Stand der Technik bekannten Biomaterialien den Vorteil, dass es einfach, modular hergestellt, in seinen Komponenten getrennt produziert, analysiert und gelagert, und individuell und spezifisch für die jeweils erwünschte Anwendung bereitgestellt werden kann.

Unter "Bioverbundmaterial" wird dabei vorliegend jedes biologische oder synthetische Material verstanden, das eine biologische Funktionalität aufweist, biologisch abbaubar ist, und mindestens zwei Komponenten aufweist. Dabei weist das Bioverbundmaterial insgesamt eine hinreichende Stabilität und Flexibilität auf, um sowohl einfach gehandhabt werden zu können, als auch um verschiedene biologische Funktionen übernehmen zu können, wie bspw. die eines Implantats, eines Arzneimittel-Freisetzenden Kissens, einer dreidimensionalen Matrix für Zellkulturen, einer bioaktiven Beschichtung für implantierbare Medizinprodukte, etc.

Unter "Trägerstruktur" wird dabei vorliegend jede Struktur verstanden, die die Funktion eines Trägers für eine in die Trägerstruktur einzubringende weitere Struktur besitzt, d.h. die als Stütze oder Gerüst für in diese Trägerstruktur einzubringende weitere Komponenten fungiert.

Unter "Matrixstruktur" wird vorliegend jedes Material verstanden, das als Aufnahmestruktur oder -material für Substanzen oder Stoffe fungiert, bzw. jedes Material, an welches Substanzen oder Stoffe gebunden werden können, sei es adhäsiv, kovalent, etc.

Unter "Wirkstoff" wird vorliegend jede Substanz verstanden, die in irgendeiner Weise eine Wirkung auf Zellen, Zellverbände oder Zellbestandteile ausübt und dort Reaktionen der Zelle oder Zellbestandteile auslösen kann. Diese Wirkung kann sich bspw. darin zeigen, dass durch den Wirkstoff in den Zellen eine bestimmte Reaktion getriggert wird, die sich wiederum auf die Zellen selber auswirkt.

Die erfindungsgemäßen Bioverbundmaterialien können bspw. in dem eingangs erläuterten Gebiet des Tissue-Engineerings eingesetzt werden, und dort bspw. als Ersatz von zelldotierten Implantaten dienen. So können die Bioverbundmaterialien nach deren Implantation an einen gewünschten Wirkort bspw. Stammzellen aus der Umgebung durch auf den Biomaterialien gebundenen und freizusetzenden Wachstumsfaktoren lokal zur Differenzierung und Proliferation anregen und damit eine gewünschte Geweberegeneration induzieren und steuern.

Ferner eigen sich die erfindungsgemäßen Bioverbundmaterialien bspw. als Implantate zur Behandlung von degenerativen Gelenkserkrankungen, wobei gegenüber derzeitigen Therapiekonzepten mit einer systemischen Verabreichung von Wirkstoffen bei der vorliegenden Erfindung der Vorteil besteht, dass die behandelten Patienten durch eine lokale, hohe, die Regeneration gezielt unterstützende Wirkstofffreisetzung einer insgesamt auf den Gesamtpatienten bezogen deutlich geringeren Wirkstoffdosis ausgesetzt sind und dadurch das Risiko unerwünschter Nebenwirkungen oder toxischer Effekte stark reduziert werden kann. V.a. bei der Verabreichung von Wachstumsfaktoren kann durch den Einsatz der erfindungsgemäßen Bioverbundmaterialien mit der damit möglichen zeitlich und räumlich exakt dosierbarer Wirkstoffapplikation bspw. eine Induktion eines Tumorwachstums in anderen Körperregionen verhindert werden.

Darüber hinaus kann das Bioverbundmaterial als dreidimensionale Matrix für die Zellkultur in der Forschung eingesetzt werden, bspw. zur Untersuchung bestimmter Zelleigenschaften oder zur Untersuchung der Effekte bestimmter, auf/in dem Bioverbundmaterial eingebundener Wirkstoffe auf Zellen. Vorteilhaft ist dabei der modulare Aufbau des Materials, der eine hohe Flexibilität und Diversität in der Anwendung im F&E Einsatz ermöglicht.

Insbesondere ist bevorzugt, wenn die Trägerstruktur ein Polymer aufweist, das ausgewählt ist aus der Gruppe der synthetischen Polymere (z.B. Polylactid, Polyglycolid, Polyethylenglycol, Polyvinylalkohol, Polyimin, Polyurethan, polyHydroxybuttersäure, Polyethylenterephthalat, Polyterafluorethylen, Polypropylen, usw.) oder der Biopolymere, wie z.B. Polypeptide/Proteine (z.B. Gelatine, Kollagen, Fibrin, usw.) oder Polysaccharide (z.B. Stärke, Zellulose, Agarose, Alginat, Dextran, Chitosan, Hyaluronsäure, usw.).

Diese Ausführungsform hat den Vorteil, dass zum Aufbau der Trägerstruktur auf Materialien zurückgegriffen werden kann, die im Bereich der regenerativen Medizin bereits erprobt sind bzw. bereits als zugelassenen Implantate eingesetzt werden und sich in diesem Bereich als geeignetes Ausgangsmaterial erwiesen haben.

Bei einer Ausführungsform des erfindungegemäßen Bioverbundmaterials ist bevorzugt, wenn die Matrixstruktur ein Polymer aufweist, das ausgewählt ist aus synthetischen Polymeren (z.B. Polyimin, Polyvinylalkohol, Polyethylenglycol, Polyurethan, Polyasparaginsäure,Polylysin, usw.) oder Biopolymeren (Gelatine, Kollagenfragmente, Albumin, Dextran, Heparansulfat, Hyaluronsäure, Chitosan, Nucleinsäuren, Peptide, Lipide, usw.) bzw. biotechnologisch hergestellten polymeren Molekülen (z.B. Aptamere, Fusionsproteine, etc.), die einen Einbau in die Trägerstruktur und eine chemische oder physikalische Anbindung von Wirkstoffen ermöglichen. Die Polymere der Matrixstruktur können dabei wiederum unterschiedliche Eigenschaften aufweisen und auch als Gemisch eingesetzt werden.

Bspw. kann Gelatine aufgrund der zur Herstellung verwendeten Ausgangsmaterialien (Schweineschwarte, Rinderspalt, Knochen) oder aufgrund der Produktionsbedingungen (alkalisch, sauer) mit unterschiedlichen chemischen und physikochemischen Eigenschaften produziert und durch weitere Fraktionierungsschritte hinsichtlich Molekulargewicht, isoelektrischem Punkt oder Hydrophobizität für die vorgesehene Aufgabenstellung als Matrixkomponente optimal selektiert werden. Hierzu können bekannte Screeningsysteme, wie z.B. die Oberflächenplasmonenresonanzspektroskopie ("Biacore-Technologie") eingesetzt werden, die eine vergleichende Messung der physikochemischen Wechselwirkung zwischen Matrixkomponente und Wirkstoff- oder Trägerkomponente ermöglichen.

Bei einer Weiterbildung des erfindungsgemäßen Bioverbundmaterials ist bevorzugt, wenn der Wirkstoff ausgewählt ist aus zumindest einer der Gruppen niedermolekulare Wirkstoffe (Entzündungshemmer, Enzyminhibitoren, Antibiotika, Hormone usw.), Wachstumsfaktoren (z.B. Bone Morphogenetic Proteine BMP-2, -7, usw. ),oder anderer Proteinwirkstoffe (bspw. Chemokine usw.) Zu den Wirkstoffen gehören auch bioaktive Peptide, wie bspw. Typ I Kollagenpeptide mit einem mittleren Molekulargewicht von ≤ 3500 D, für das eine stimulierende Wirkung auf Chondrozyten nachgewiesen wurde.

Insbesondere ist bevorzugt, wenn der Wirkstoff durch eine unspezifische Bindung an die Matrixstruktur in das Biomaterial eingelagert ist.

Das Einbringen des Wirkstoffs sowie die Bindung an die Matrixstruktur kann optimiert erfolgen durch die Verwendung von Mediatoren - Komponenten, die sowohl den Wirkstoff effektiv solubilisieren als auch eine hohe Kompatibilität zur Matrix aufweisen. Im Fall von unlöslichen Gelatinematrizes eignen sich zu diesem Zweck lösliche niedermolekulare Gelatinefragmente.

Diese Ausführungsform hat den Vorteil, dass der einzubringende Wirkstoff bspw. lediglich durch bloßes Mischen mit der Matrixstruktur über physikochemische Wechselwirkungen sehr schonend an die Matrixstruktur gebunden und zusammen mit der Matrixstruktur in die Trägerstruktur aufgenommen wird. Der so in dem Bioverbundstoff vorliegende Wirkstoff wird daher nicht modifiziert und kann bspw. bei einem Einsatz zum Zwecke der Geweberegeneration nach Implantation einfach aus dem Biomaterial freigesetzt werden, ohne dass der Wirkstoff aktiv durch weitere Maßnahmen aus dem Biomaterial gelöst werden muss. Die Steuerung der Wirkstofffreisetzung erfolgt hierbei 1. über die Gleichgewichtskonstante der Bindung von Wirkstoff und Matrixstruktur, die über einfaches Interaktionsscreening bestimmt werden kann, und 2. durch den Diffusionseffekt im Träger. Ein weiterer Vorteil dieser Ausführungsform ist die einfache Herstellungsprozedur des Verbundmaterials, die eine schnelle Methodenoptimierung und eine günstige Produktion unter regulatorischen Gesichtspunkten ermöglicht.

In einer weiteren Ausführungsform ist bevorzugt, wenn der Wirkstoff über Bindemoleküle, insbesondere Peptide, die den Wirkstoff spezifisch binden, an die Matrixstruktur gebunden wird und als Wirkstoff-Matrix in das Biomaterial eingelagert werden kann.

Diese Ausführungsform hat den Vorteil, dass die Wirkstoffe gezielt über Bindemoleküle, die die Wirkstoffe spezifisch, in der bevorzugten Form in einer stabilen, bioaktiven Konformation ,binden, in die Matrixstruktur und damit in das Bioverbundmaterial eingebracht werden können. Dabei werden insbesondere bereits im Stand der Technik bekannte Bindungen zwischen Wirkstoffen und Peptiden ausgenutzt, wie bspw. die Bindung von BMP-2 (Bone Morphogenetic Protein 2) an bestimmte Zyklopeptide (Behnam, K., Phillips, M. L., Silva, J. D., Brochmann, E. J., Duarte, M. E., and Murray, S. S. (2005). BMP binding peptide: a BMP-2 enhancing factor deduced from the sequence of native bovine bone morphogenetic protein/non-collagenous protein. J Orthop Res 23, 175-180..)

Es versteht sich, dass bei dieser Ausführungsform des Bioverbundmaterials jedes Bindemolekül eingesetzt werden kann, das eine spezifische Bindung von Wirkstoffen und deren Freisetzung bspw. über die Zeit unter physiologischen Bedingungen ermöglicht. Das "Bindemolekül" kann dabei bspw. ein Rezeptor, ein Cofaktor oder ein Protein der extrazellulären Matrix sein. Außerdem können dafür auch Aptamere, Antikörper, Antikörperfragmente, Affibodies bzw. andere Bindemoleküle mit geeigneter Bindungskonstante und Affinität spezifisch generiert werden.

Bei einer Weiterbildung des erfindungsgemäßen Bioverbundmaterials ist dabei bevorzugt, wenn die Zyklopeptide wiederum über Linker kovalent an die Matrixstruktur gebunden sind. Hierbei ist bevorzugt, wenn die Anbindung des Linkers über Reaktionen der Reaktionspartner Thiol, wie bspw. im Cystein, und Maleimid, oder N-Hydroxylamin und Aldehyd, Amin-Aldehyd erfolgt, oder anderen spezifischen im Stand der Technik bekannten Kopplungsverfahren, und die bspw. unter physiologischen Bedingungen gebildet werden können.

Bei bestimmten Wirkstoffen kann die Wirkung bspw. durch Bindemoleküle, die selbst als Cofaktoren biologisch aktiv sind, bei dieser Ausführungsform noch gesteigert werden. In diesen Fällen wird die biologische Aktivität des Wirkstoffes bei Freisetzung von der Matrixstruktur geringer. Solche Verbundmaterialien ermöglichen die vorteilhafte Reduktion der therapeutischen Wirkstoffmenge und bieten eine verbesserte pharmakologische Sicherheit, da dadurch potentielle Nebenwirkungen minimiert werden.

In eigenen Versuchen konnten die Erfinder zeigen, dass BMP-2, das über verschiedene Zyklopeptide an die Matrixstruktur und damit in dem Biomaterial gebunden war, über einen Zeitraum von mehr als drei Wochen seine Aktivität im Träger beibehielt, mit Zellen interagierte und kaum in die Umgebung des Trägers abgegeben wurde.

Die Erfindung betrifft daher insbesondere ein Bioverbundmaterial für die kontrollierte Freisetzung und Applikation von Wirkstoffen, umfassend eine dreidimensionale Trägerstruktur auf Polymerbasis, sowie eine Matrixstruktur auf Polymerbasis, welche in die dreidimensionale Trägerstruktur eingebracht ist, wobei der Wirkstoff über Peptide an die Matrixstruktur gebunden ist, welche die Wirkstoffe spezifisch binden.

Insbesondere ist in bestimmten Ausführungsformen bevorzugt, wenn der Wirkstoff der Wachstumsfaktor BMP-2 (Bone Morphogenic Protein 2) ist, der über für BMP-2 spezifische Zyklopeptide spezifisch gebunden ist. Insbesondere ist bevorzugt, wenn das Peptid, das den Wirkstoff spezifisch bindet, von einem Protein abgeleitet ist, das ausgewählt ist aus bovinem Fetuin, bovinem BMP-binding Protein (BBP) oder dem Rezeptor für den transformierenden Wachstumsfaktor β (TGFβR-2).

In eigenen Versuchen konnten die Erfinder zeigen, dass mit Peptiden, die Sequenzen der genannten Proteine aufweisen, BMP-2 effektiv an die Matrixstruktur gebunden werden kann, wobei gleichzeitig eine effektive Freisetzung des Wirkstoffs in die Umgebung über einen längeren Zeitraum nachgewiesen werden konnte. Überraschenderweise konnten die Erfinder auch zeigen, dass die biologische Aktivität des Wachstumsfaktors durch die Zyklopeptide sogar gesteigert wurde.

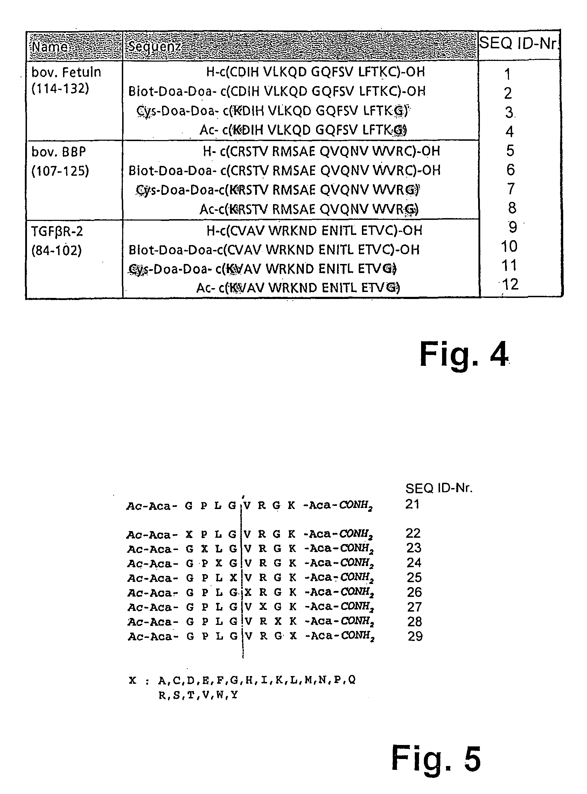

Dabei ist insbesondere bevorzugt, wenn das Peptid, das den Wirkstoff spezifisch bindet, eine Sequenz aufweist, die ausgewählt ist aus einer der SEQ ID Nr. 1 bis 12 aus Fig. 4 und dem beigefügten Sequenzprotokoll.

Die aufgeführten Peptide konnten stabil zyklisiert und über einen Spacer und einen Linker an die Matrixstruktur immobilisiert werden, wodurch die Interaktionen zwischen immobilisiertem Zyklopeptid und BMP-2 untersucht werden konnten.

Dabei zeigte sich eine deutliche konzentrationsabhängige Bindung an bzw. Freisetzung des BMP-2 vom Zyklopeptid.

Bei einer noch weiteren Ausführungsform ist bevorzugt, wenn der Wirkstoff kovalent an die Matrixstruktur gebunden ist, und insbesondere wenn der Wirkstoff über Linkermoleküle an die Matrixstruktur gebunden ist, welche enzymatisch, insbesondere z.B. durch eine Protease, Glycariase, Nuclease usw. spaltbar sind.

Daher betrifft die Erfindung auch Bioverbundmaterialien für die kontrollierte Freisetzung von Wirkstoffen, umfassend eine dreidimensionale Trägerstruktur auf Polymerbasis, sowie eine Matrixstruktur auf Polymerbasis, welche in die dreidimensionale Trägerstruktur eingebracht ist, wobei der Wirkstoff über kovalent an die Matrixstruktur gebundene Linkermoleküle an die Matrixstruktur gebunden ist, wobei die Linkermoleküle durch Enzyme, bspw. durch Proteasen, spaltbar sind.

Bei dieser Maßnahme wird vorteilhafterweise die Umgebung ausgenutzt, in welche das Bioverbundmaterial bspw. in Form eines Implantats eingeführt wird, bzw. die enzymatischen Gegebenheiten der Umgebung, wonach die Freisetzung der Wirkstoffe von der vorhergesagten oder berechneten Aktivität bspw. zelleigener Proteasen abhängig gemacht werden kann.

Insbesondere ist dabei bevorzugt, wenn die Peptide durch eine krankheitsassoziierte Matrixmetalloprotease MMP (z.B. MMP-1, 3, 7, 8, 9, 10, 13 usw.) spaltbar sind. Matrixmetalloproteinasen sind Mitglieder der großen Familie der Zink-abhängigen Endopeptidasen, die wesentlich an dem Abbau der extrazellulären Matrix beteiligt sind. Sie werden von einer Vielzahl von Zellen gebildet und als sog. Zymogene sezerniert. Die Aktivität der von den Zellen sezernierten MMPs wird dann ausgenutzt, um die MMP-sensitiven Peptide, über die die Wirkstoffe im Biomaterial gebunden sind, zu spalten und so die Wirkstoffe freizusetzen.

In eigenen Versuchen konnte die erfolgreiche Freisetzung von Substanzen durch eine MMP-7-Aktivität mit verschiedenen Peptid-Substraten gezeigt werden.

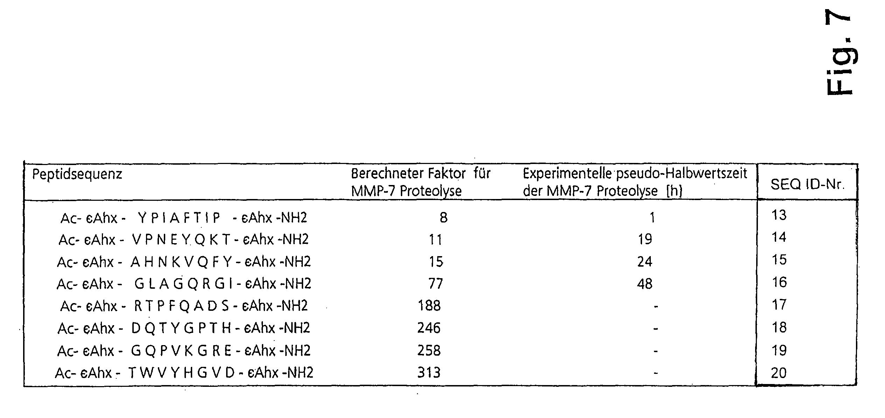

Dabei ist insbesondere bevorzugt, wenn das an die Matrixstruktur gebundene Peptid eine Sequenz aufweist, die ausgewählt ist aus einer der SEQ ID Nr. 13 bis 20 aus Fig. 7 und dem beigefügten Sequenzprotokoll.

Wie weiter oben erwähnt, betrifft die Erfindung auch ein Verfahren zur Herstellung eines Bioverbundmaterials, umfassend eine dreidimensionale Trägerstruktur auf Polymerbasis, sowie eine Matrixstruktur auf Polymerbasis, mit den folgenden Schritten:
a) Bereitstellen von i) Trägermaterial auf Polymerbasis und ii) Matrixmaterial auf Polymerbasis;
b) ggf. Hydrophilisieren des Trägermaterials; und
c) Einbringen des Matrixmaterials in das hydrophilisierte Trägermaterial aus Schritt b) zur Herstellung eines Bioverbundmaterials mit einer Trägerstruktur und einer darin eingebrachten Matrixstruktur.

Diese Maßnahme hat den Vorteil, dass das Trägermaterial und das Matrixmaterial separat vorbereitet werden kann, weshalb es möglich ist, beide Materialien, oder auch nur eines davon, auf Vorrat herzustellen und stabil ggf. unter verschiedenen vorteilhaften Bedingungen zu lagern. Hierbei ist vorteilhaft, dass Herstellungsprozesse von Trägermaterial und Matrix-Wirkstoffmaterial getrennt erfolgen und damit für sich optimierbar sind. Hinsichtlich einer Zulassung der Verbundmaterialien als Medizin- oder Pharmaprodukt können die einzelnen Prozesse getrennt validiert und dokumentiert und eine ggf. erforderliche Reinigung der Matrix-Wirkstoffmaterialien von Chemikalien aus dem Herstellungsprozess mit etablierten Methoden der pharmazeutischen Produktion (z.B. Chromatographie) sichergestellt werden. Eine entsprechende Reinigung ist in dreidimensionalen Trägerstrukturen nicht durchführbar.

Ferner ist vorteilhaft, dass bspw. das Matrixmaterial vor Einbringung in das Trägermaterial spezifisch für die jeweils erwünschte Verwendung modifiziert wird, bspw. durch eine Wirkstoffdotierung des Matrixmaterials, d.h. durch Einbringen des Wirkstoffs in das Matrixmaterial durch Diffusion, ferner bspw. durch Modifizierung mit Peptiden, die bestimmte Wirkstoffe wiederum spezifisch erkennen und binden, oder aber durch proteaselabile Peptide und daran gebundene Wirkstoffe. Durch diese separate Bereitstellung wird daher auch eine individuelle Zusammenstellung des Bioverbundmaterials ermöglicht. Demnach kann die Auswahl des Trägermaterials und des Matrixmaterials an verschiedene Applikationen adaptiert werden, ebenso wie die Auswahl bzw. Herstellung selektiver Bindemoleküle, oder geeigneter Proteasesubstrate. Darüber hinaus kann bei einer Bindung der Wirkstoffe über selektive Bindungsmoleküle die Dissoziationskonstante zur Vorhersage der Freisetzungskinetik bestimmt werden, sowie bei einer Bindung über proteaselabile Peptide die Freisetzungsdauer von der gemessenen oder erwarteten Proteaseaktivität am Wirkort abhängig gemacht werden.

Es können ferner vorteilhafterweise auch mehrere verschiedene Wirkstoffe in das Bioverbundmaterial eingebunden werden, und sogar deren jeweilige Freisetzungskinetik individuell eingestellt werden. Ggf. kann die Zugabe des Wirkstoffs auch unmittelbar vor einem Einsatz des Bioverbundmaterials erfolgen, so dass ein vorzeitiger Abbau der Wirkstoffe und damit deren vorzeitige Unwirksamkeit vermieden wird.

Insbesondere ist bei dem erfindungsgemäßen Verfahren bevorzugt, wenn das Trägermaterial und/oder das Matrixmaterial ein Material wie weiter oben für das Bioverbundmaterial beschrieben aufweist.

Die hierbei verwendeten Gelatineträger können z.B entsprechend WO 2005 111 121 A2 hergestellt werden.

Bei dem erfindungsgemäßen Verfahren ist ferner bevorzugt, wenn der Wirkstoff ausgewählt ist aus einer der Gruppen niedermolekulare Wirkstoffe (Entzündungshemmer, Enzyminhibitoren, Antibiotika, Hormone usw.), Wachstumsfaktoren (z.B. Bone Morphogenetic Proteine BMP-2, -7, usw.), oder anderer Proteinwirkstoffe (bspw. Chemokine usw.) Insbesondere ist bevorzugt, wenn der Wirkstoff durch eine unspezifische Bindung an die Matrixstruktur in das Biomaterial eingelagert ist.

Die vorliegende Erfindung betrifft daher auch die Verwendung eines erfindungsgemäßen Bioverbundmaterials, oder eines Bioverbundmaterials, das nach dem erfindungsgemäßen Verfahren hergestellt ist, zur Herstellung eines Arzneimittels für die Behandlung von degeneriertem oder erkranktem Gewebe.

Vorteilhafterweise kann also das erfindungsgemäße Bioverbundmaterial, das mit entsprechenden Wirkstoffen, bspw. BMP-2 beladen ist, zur Behandlung von Gelenkschäden und zur Regeneration von Knorpel eingesetzt werden. Durch die lokale, auf das Bioverbundmaterial begrenzte Abgabe von entsprechenden Wirkstoffen wie Wachstumsfaktoren können Zellen bzw. Gewebe, das das implantierte Bioverbundmaterial umgibt, gezielt beeinflusst werden, wie bspw. Stammzellen zur Differenzierung und Proliferation und damit zur Neubildung von Gewebe. Die lokale Freisetzung von Wachstumsfaktoren in einem Träger ist besonders vorteilhaft, da dadurch hohe Wirkkonzentrationen bei geringsten systemischen Konzentrationen der Wirkstoffe erreicht werden können. Dadurch können unerwünschte Nebenwirkungen, die bspw. bei Einsatz von Wachstumsfaktoren in der Induktion von Tumoren bzw. Erhöhung der Tumorzellproliferation bestehen können, vermieden werden.

Das erfindungsgemäße Bioverbundmaterials kann aber auch als Arzneimittel eingesetzt werden, mit dem lokal Chemotherapeutika an das das Bioverbundmaterial umgebende Gewebe oder an die Zellen abgegeben werden sollen. Vorteilhafterweise werden dabei die auf/in dem Bioverbundmaterial eingebundenen Wirkstoffe durch eine gezielt eingestellte Freisetzungskinetik, bspw. über eine Protease-Aktivität, in die Umgebung abgegeben. Dadurch lassen sich bspw. gezielt und spezifisch Krebszellen behandeln, so dass eine systemische Verabreichung von Chemotherapeutika, die in der Regel mit vielen Nebenwirkungen und Belastungen für den Körper eines Patienten verbunden sind, vorteilhaft vermieden werden.

Ferner betrifft die Erfindung die Verwendung des erfindungsgemäßen Bioverbundmaterials oder eines Bioverbundmaterials, das nach dem erfindungsgemäßen Verfahren hergestellt ist, für die Zellkultur.

Bei dieser Verwendung wird somit das Bioverbundmaterial als dreidimensionale Matrix eingesetzt, die mit den jeweils erwünschten Wirkstoffen beladen ist und auf die Zellen ausgesät werden. Dadurch können einerseits die Effekte bestimmter Wirkstoffe - alleine oder in Kombination mit anderen Wirkstoffen - auf unterschiedliche Zellen bzw. Zellarten untersucht werden. Vorteil dieses Bioverbundmaterials ist das modulare Konzept, das eine schnelle und effiziente Zusammenstellung unterschiedlicher Wirkstoff-Matrix Kombinationen mit verschiedenen Trägerstrukturen, - porositäten usw. ermöglicht.

Weitere Vorteile ergeben sich aus den Figuren und der nachstehenden Beschreibung.

Die Erfindung wird anhand der nachfolgenden Figuren und den Beispielen weiter erläutert. Es zeigen
- Fig. 1: eine schematische, ausschnittsweise Darstellung des Aufbaus des erfindungsgemäßen Bioverbundmaterials gemäß einer ersten Ausführungsform;
- Fig. 2: eine schematische, ausschnittsweise Darstellung des Aufbaus des erfindungsgemäßen Bioverbundmaterials gemäß einer zweiten Ausführungsform, bei der die Wirkstoffe über spezifische Bindemoleküle in das Biomaterial aufgenommen sind;
- Fig. 3: eine schematische, ausschnittsweise Darstellung des Aufbaus des erfindungsgemäßen Bioverbundmaterials gemäß einer dritten Ausführungsform, bei der die Wirkstoffe über spezifische, enzym-labile Linker in das Biomaterial aufgenommen sind;
- Fig. 4: die tabellarische Auflistung von beispielhaft verwendeten zyklischen Peptiden für spezifische BMP-2 Bindung entsprechend der zweiten Ausführungsform.
- Fig. 5: eine Übersicht der Peptidbibliothek die zur Identifizierung der ProteaseSensitivität (MMP-7) verschiedener Peptidsequenzen eingesetzt wurde,
- Fig. 6: eine tabellarische Übersicht über die Inkremente der PseudoHalbwertszeit der Proteolyse (MMP 7), die aus Untersuchungen der Abbaukinetiken der Peptidbibliotheken aus Fig. 5 bestimmt wurden; und
- Fig. 7: eine tabellarische Übersicht des Vergleichs der aus den Inkrementen in Fig. 6 berechneten Faktoren für MMP-7-Proteolyse von Oktapeptiden mit den experimentell bestimmten Pseudo-Halbwertszeiten der Proteolyse.

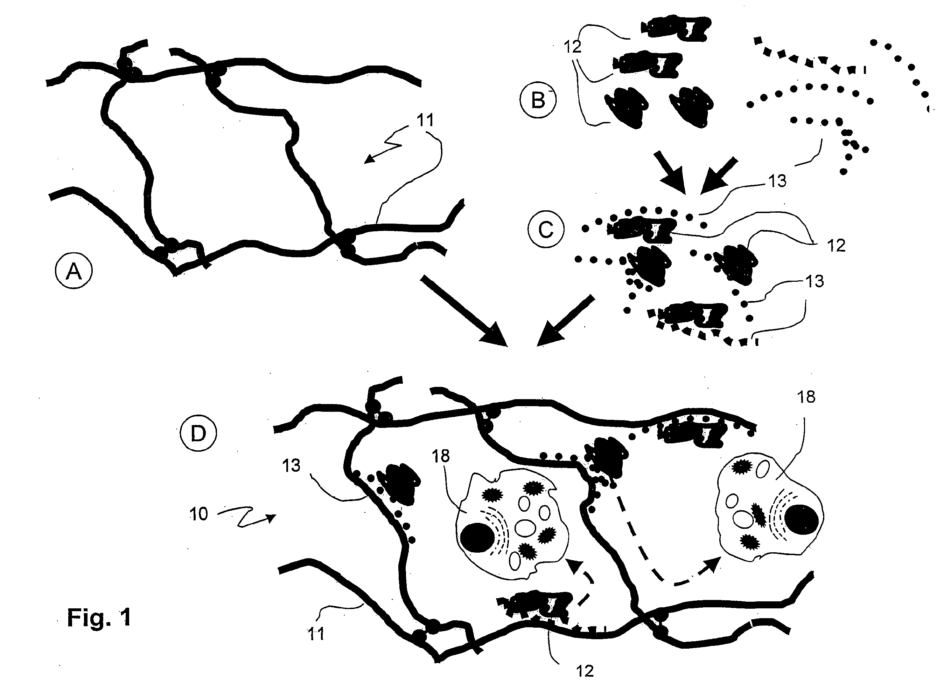

In Fig. 1 ist mit 10 insgesamt ein Bioverbundmaterial bezeichnet, das aus einer Trägerstruktur 11, einer Matrixstruktur 13 und Wirkstoffen 12 modular aufgebaut ist. In (B) sind die Wirkstoffe 12 und die Matrixstruktur 13 bereitgestellt und in (C) gemischt, wodurch über physikochemische Wechselwirkungen Matrix-Wirkstoffkomplexe generiert werden, die anschließend mit der in (A) dar- und bereitgestellten dreidimensionalen Trägerstruktur 11 kombiniert werden (D).

Die Wirkstoffe 12 können dabei bspw. Wachstumsfaktoren sein, die aus dem Bioverbundmaterial 10 freigesetzt werden. Die Wirkstoffe 12 gelangen dann in Kontakt mit Zellen 18, die, bei einer Implantation in ein menschliches oder tierisches Gewebe, entweder in das Bioverbundmaterial einwandern oder dieses umgeben und dadurch in Kontakt mit den freigesetzten Wirkstoffen/Wachstumsfaktoren kommen. Auf der anderen Seite können die Zellen 18, bei zellbesiedelten Implantaten oder bei einer Verwendung des Bioverbundmaterials 10 als dreidimensionale Matrix für die Zellkultur, auch einfach auf dem Bioverbundmaterial 10 ausgesät werden, wodurch sie auch hier in Kontakt mit den freizusetzenden Wirkstoffen kommen.

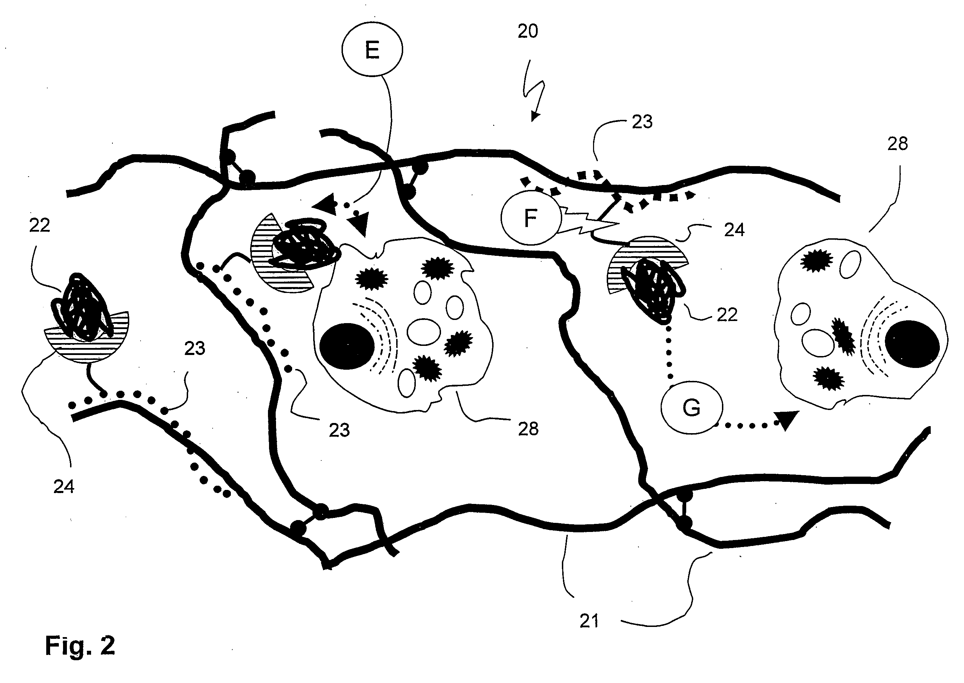

In Fig. 2 ist eine weitere Ausführungsform eines erfindungsgemäßen Bioverbundmaterials 20 gezeigt, bei welchem Wirkstoffe 22 über Bindemoleküle 24, bspw. Peptide, die kovalent an Matrixkomponenten 23 gekoppelt sind , in eine dreidimensionale Trägerstruktur 21 eingebracht werden. Die Wirkstoffe 22 werden mit der Zeit und/oder unter physiologischen Bedingungen freigesetzt (F) und wirken dann auf die das Bioverbundmaterial umgebenden, bzw. auf die in dieses eingewanderten oder auf dieses ausgesäte Zellen 28. Alternativ können die an Bindemoleküle 24 gebundenen Wirkstoffe 22 auch ohne vorherige Freisetzung die Zellen in direkter Nachbarschaft beeinflussen (E) oder von den Bindemolekülen abdiffundieren und mit Zellen 28 interagieren (G).

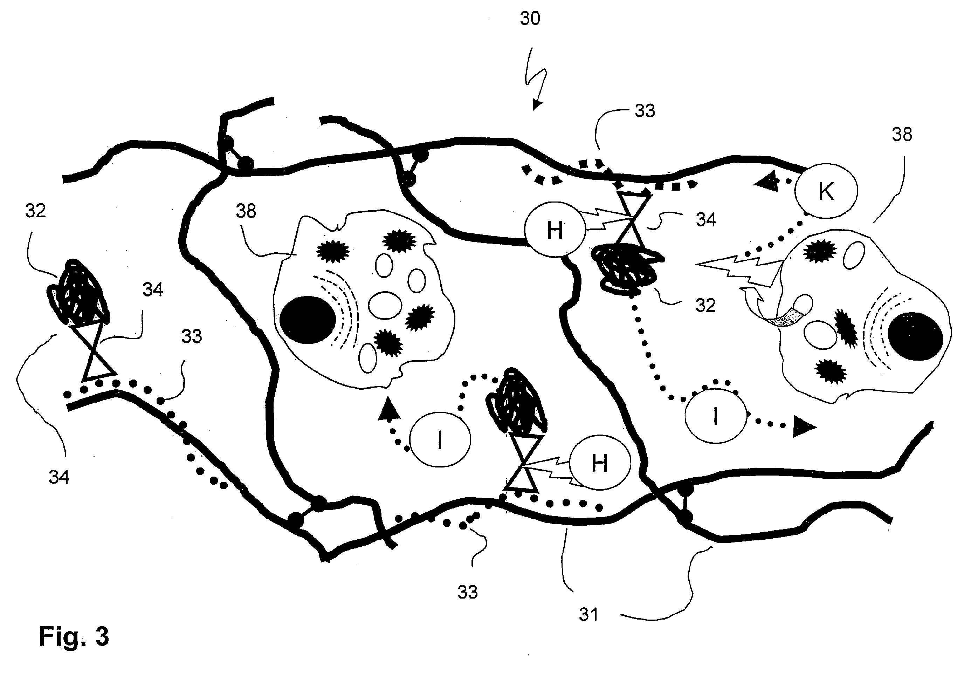

In Fig. 3 ist mit 30 insgesamt noch eine weitere Ausführungsform eines erfindungsgemäßen Bioverbundmaterials gezeigt. Hier sind die Wirkstoffe 32, bspw. Wachstumsfaktoren, über Enzym-spaltbare Linkermoleküle 34 an eine Matrixstruktur 33 kovalent gekoppelt, die an die dreidimensionale Trägerstruktur 31 gebunden sind. Die Wirkstoffe 32 werden durch eine enzymatische Spaltung der Linkermoleküle (H) von der Matrixstruktur und der Trägerstruktur freigesetzt, wodurch sie mit Zellen 38 in Wirkung treten können (I). Die Enyzme, bspw. Proteasen, werden dabei von den Zellen, die das Biomaterial *in vivo* oder *in vitro* umgeben, sezerniert (K).

In Fig. 4 sind Peptidsequenzen angegeben, die für die spezifische Bindung von BMP-2 an polymere Träger verwendet wurden. Die Peptide wurden aus Sequenzen nach Stand der Technik abgeleitet (Behnam, K., Phillips, M. L., Silva, J. D., Brochmann, E. J., Duarte, M. E., and Murray, S. S. (2005). BMP binding peptide: a BMP-2 enhancing factor deduced from the sequence of native bovine bone morphogenetic protein/non-collagenous protein. J Orthop Res 23, 175-180.). Es wurden zyklische Peptide über Ausbildung von Disulfidbrücken zwischen Cysteinresten (C) (no. 1,2 jeder Serie) oder durch Ausbildung einer Peptidbindung zwischen C-Terminus und der ε-Aminogruppe von terminalem Lysin (K) (no. 3,4 jeder Serie) hergestellt. Die Peptide wurden als freie (no. 1 jeder Serie) bzw. acetylierte Peptide (no. 4 jeder Serie) mit Spacern (Doa : 3,6-Dioxa-8-aminooctansäure) und Biotin (no. 2 jeder Serie) bzw. Cystein (no. 3 jeder Serie) für die Anbindung an Streptavidin bzw. Maleimidogruppen synthetisiert.

In Fig. 5 ist der schematische Aufbau der Peptidbibliotheken dargestellt, mit denen die pseudo-Halbwertszeiten der Proteasespaltung bestimmter Sequenzmotive in Peptiden bestimmt wurden. X entspricht den 20 proteinogenen Aminosäuren. Die parallele Quantifizierung der Spaltfragmente und der intakten Peptide erfolgte durch HPLC-Elektrospray-Massenspektrometrie.

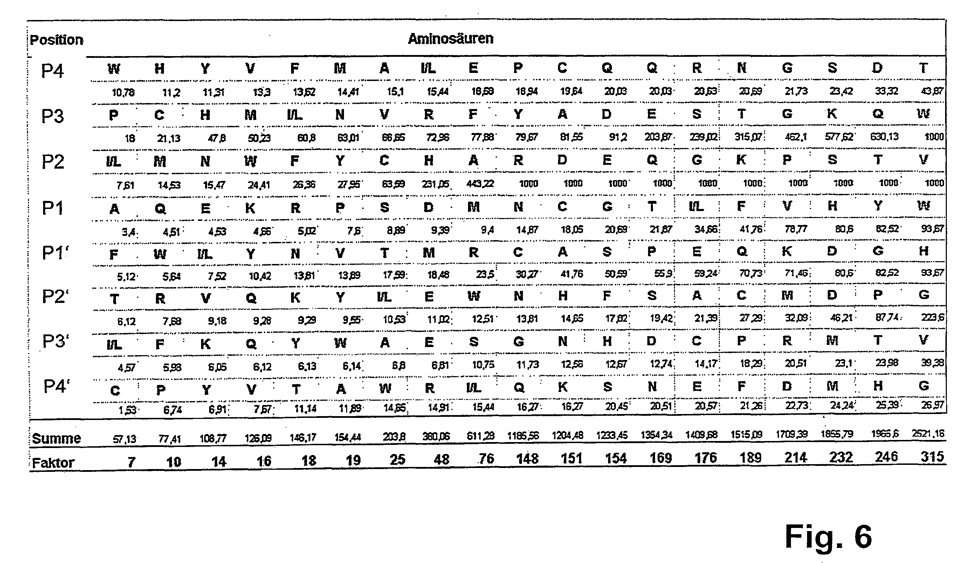

In Fig. 6 sind die Ergebnisse der in Fig. 5 beschriebenen Experimente zusammengefasst. Wenn nach 24 h keine Spaltung der Peptide detektierbar war wurde die Pseudo-Halbwertszeit für diese Aminosäure an der entsprechenden Position in der Sequenz auf 1000 gesetzt. Zur Vorhersage der relativen Proteaselabilität einer Peptidsequenz werden alle Pseudo-Halbwertszeiten der Aminosäuren in der entsprechenden Position zur Spaltstelle aufsummiert und durch die Zahl der Positionen (im Beispiel 8 für P4 - P4') dividiert. Je kleiner der so berechnete Faktor desto schneller wird die entsprechende Peptidsequenz durch die Protease gespalten. Im Beispiel sind die Werte für MMP-7 gezeigt.

Fig. 7 zeigt Beispiele für Peptidsequenzen, die aus der Tabelle in Fig. 6 für MMP-7 Substrate abgeleitet wurden. Die berechneten Faktoren für die Proteolyse mit MMP-7 werden mit den experimentell bestimmten Pseudo-Halbwertszeiten verglichen. Es zeigte sich eine gute Korrelation zwischen Vorhersage und experimentell bestimmter Proteaselabilität.

### Beispiele

### 1.) Unspezifische Bindung

### A) Einbringen von hochpolymeren Substanzen in poröse dreidimensionale Strukturen von Gelatineträgern.

Die Versuche wurden mit neu entwickelten Gelatineträgern (Herstellung entsprechend WO 2005 111 121 A2) durchgeführt. Durch eine einfache Plasmabehandlung mit Sauerstoff wurden die Träger hydrophilisiert. In die hydrophilisierten Träger wurden wasserlösliche Polymere (Matrix) wie z.B. kurzkettige Gelatinefraktionen und Wirkstoffe wie BMP-2 innerhalb weniger Sekunden in den Träger eingebracht, und verteilten sich gleichmäßig darin. Die homogene Verteilung der aufgenommenen Polymere in der Trägerstruktur konnte mittels fluoreszenzmarkierter (Cy3)-Gelatine getestet werden. Die Homogenität der Verteilung und die Anbindung der hochmolekularen Matrixstrukturpolymere im Träger wurde dadurch nachgewiesen.

### B) Bindungskinetik von BMP-2 und verschiedenen Gelatinetypen

Surface-Plasmonen-Resonanz-Analysen mit immobilsiertem BMP-2 und unterschiedlichen gelösten Gelatineproben zeigten Unterschiede in der Bindung der verschiedenen Gelatinen an den Wachstumsfaktor. Eingesetzt und getestet wurde PS-Gelatine (Typ A), Sol-LDA Gelatine (Typ A), LB-Gelatine (Typ B) und Sol-D Gelatine (Typ B) (Gelita AG, Eberbach, D)):

| Name | Typ | IP | Gelst. |
|---|---|---|---|
| PS | A | ca. 9 | 300 g Bloom |
| Sol LDA | A | ca. 9 | 0 |
| LB | B | ca. 5 | 315 g Bloom |
| Sol D | B | ca. 5 | 0 |

| | | | |
|---|---|---|---|
| (IP = isoelektrischer Punkt; A = saure, B = basische Hydrolyse; Gelst. = erreichbare Gelstärken) | | | |

Es zeigte sich, dass die Bindung von hochpolymerer Gelatine stärker war als die Bindung von niedermolekularer Gelatine. Ferner konnte gezeigt werden, dass basische Gelatine (IP 9, PS, Sol LDA) stärker bindet als saure Gelatine (IP 5, LB, Sol D). BSA (Rinderserumalbumin) wurde als Kontrolle eingesetzt.

### C) Gelatineträger mit Wachstumsfaktoren

Entsprechend zu A) wurden Gelatineträger mit dem Wachstumsfaktor Bone morphogenetic Protein 2 (BMP-2) dotiert. Die so dotierten Träger wurden unterschiedlich lange in Zellkulturmedium unter Kulturbedingungen gelagert. Anschließend wurde der Überstand des Kulturmediums abgenommen und eine Zellsuspension mit pluripotenten Maus-Fibroblasten (MC3T3-E1) zugegeben, die sich nach Aktivierung mit BMP-2 zu Osteoblasten differenzieren und alkalische Phosphatase exprimieren, deren Aktivität als Maß für die Stimulation durch den Wachstumsfaktor herangezogen wurde.

Die mit diesen Versuchen gewonnenen Ergebnisse zeigen, dass der Wachstumsfaktor im Gelatineträger bis zu 28 Tagen aktiv war.

### 2. Spezifische Bindung

Aus Behnam et al. (J. Orthop. Res. (2005) 23:175-180) sind BMP-2-bindenden Peptidsequenzen bekannt. Diese zyklischen Peptide wurden mit Linkern modifiziert, über die eine gerichtete kovalente chemische Anbindung an Polymere möglich ist. Die spezifische Bindung von BMP-2 an diese Peptide konnte entsprechend der oben zitierten Literaturstelle mittels Oberflächenplasmonenresonanzspektroskopie (SPR, Biacore) gezeigt werden.

### A) Herstellung von zyklischen BMP-2 Bindepeptiden

Die in der Literatur genannten Zyklopeptide wurden synthetisiert und zusätzlich mit einem Linker (Biotin, Cystein) modifiziert. In Fig. 4 ist eine tabellarische Übersicht über die hergestellten Peptide gezeigt. Die Peptide wurden dabei nicht nur über intramolekulare Disulfidbrücken zyklisiert, sondern auch durch eine Peptidbindung zwischen C-terminalem Glycin und der ε-Aminogruppe im N-terminalen Lysin. Dadurch kann das Zyklopeptid über einen Spacer und ein N-terminales freies Cystein als Thioether kovalent an eine maleimidomodifizierte Matrixstruktur gebunden werde. Die über Disulfidbrücken zyklisierte Peptide konnten über einen Spacer und Biotin an Streptavidinoberflächen gebunden werden. Mit diesen Modellsystemen konnten Versuche zur BMP-2 Bindung und Freisetzung durchgeführt werden.

### B) Spezifische Interaktion zwischen immobilisierten Zyklopeptiden und dem Wachstumsfaktor BMP-2

Zur Bestimmung der spezifischen Interaktion zwischen den in Fig. 4 gezeigten Zyklopeptiden und BMP-2 wurden SPR Analysen durchgeführt. BMP-2 wurde dazu auf einem Biacore Chip kovalent immobilisiert und Bindungskinefiken mit unterschiedlich konzentrierten Lösungen der Zyklopeptide durchgeführt. Die hierdurch gewonnenen Ergebnisse zeigen eine deutliche konzentrationsabhängige Bindung bzw. Freisetzung des Peptids vom immobilisierten Wachstumsfaktor.

Diese Ergebnisse haben ferner gezeigt, dass die biologische Aktivität von BMP-2 durch Zugabe von zyklischen Peptiden gesteigert wird. Hierzu wurden Versuche mit pluripotenten Maus-Fibroblasten (MC3T3-E1) und Peptidgebundenem BMP-2 durchgeführt, die sich nach Aktivierung mit BMP-2 zu Osteoblasten differenzieren und alkalische Phosphatase (ALP) exprimieren. Die Aktivität der ALP wurde als Maß für die Stimulation durch den Wachstumsfaktor herangezogen. Eingesetzt wurde dabei im Beispiel das aus Fetuin abgeleitete Bindepeptid aus Fig. 4.

Für die Versuchsdurchführung wurden 500 µl MC3T3-Zellsuspension pro Well in eine 24-Well-Platte pipettiert, die Zellen anschließend 4 Stunden adhäriert und dann mit unterschiedlichen Mischungen (1 ml/Well) inkubiert und dreifach getestet. Nach 72 Stunden wurde die ALP-Aktivität gemessen. Die verwendeten Mischungen waren:

### Mischung A: BMP-2 + Bindepeptid

0,15 µg BMP-2/ml Medium (Endkonzentration im Well: 0,1 µg BMP-2/ml Medium)
3,0 µg Peptid/ml Medium (Endkonzentration im Well: 2,0 µg Peptid/ml Medium)
105 µl Peptidstammlösung + 5,25 µl BMP-2 Stammlösung Inkubation 15 min bei Raumtemperatur. Zu BMP-2/Peptid wurden 3390 µl MC3T3-E1 Komplett-Medium zugegeben.

### Mischung B: Reines BMP-2

0,15 µg BMP-2/ml Medium (Endkonzentration im Well: 0,1 µg BMP-2/ml Medium)
105 µl PBS (pH 5,6) + 5,25 µl BMP-2 Stammlösung
Inkubation 15 min bei Raumtemperatur. Zu BMP-2/Peptid wurden 3390 µl MC3T3-E1 Komplett-Medium zugegeben.

### Mischung C: Reines Peptid

3,0 µg Peptid/ml Medium (Endkonzentration im Well: 2,0 µg Peptid/ml Medium)
105 µl Peptidstammlösung + 5,25 µl PBS (pH 5,6)
Inkubation 15 min bei Raumtemperatur. Zu BMP-2/Peptid wurden 3390 µl MC3T3-E1 Komplett-Medium zugegeben.

### Mischung D: Reines C3T3-E1 Komplett-Medium (Kontrolle ohne BMP-2/Peptid)

3,5 ml MC3T3-E1 Komplett-Medium

Zu allen Ansätzen wurde anschließend 1 ml Medium mit BMP-2 zugefügt, und die Ansätze wie erwähnt für 72 Stunden inkubiert. Anschließend wurde die ALP-Aktivität gemessen. Es zeigte sich, dass der ALP-Wert bei Inkubation der Zellen mit BMP-2 und Peptid höher war als bei Inkubation mit BMP-2 alleine.

### 3.) kovalente Bindung über proteaselabile Linkerpeptide

In Vorversuchen wurden Peptidsequenzen untersucht und identifiziert, die durch Matrixmetalloproteasen (MMP) unterschiedlich schnell abgebaut werden können.

### A) Identifizierung möglichst spezifischer MMP-Peptidsubstrate

Aus bekannten MMP-Peptidsubstraten mit moderater Proteolysekinetik wurden Peptidbibliotheken mit jeweils einer randomisierten Aminosäurenposition (19 Aminosäuren) abgeleitet (siehe Fig. 5) und synthetisiert. Aus dem Substrat mit einer pseudo-Halbwertszeit im Assay von 169 wurde durch Ersetzen jeweils einer Aminosäure durch eine Mischung der 20 proteinogenen Aminosäuren eine Peptidbibliothek hergestellt, die jeweils unterschiedlich lange mit MMP 7 inkubiert wurde. Die entstandenen Proteolysefragmente wurden anschließend mittels HPLC-ESI-MS (Hochleistungs-Flüssigchromatographie-Massenspektrometrie mit Elektrospray - Ionisierung) semiquantitativ erfasst. Aus den Analysen der Verdaue wurde für jede Aminosäurenposition bezogen auf die Enzymspaltstelle und für jede Aminosäure in dieser Position ein Inkrement berechnet (siehe Fig. 6). Diese Inkremente können zur Vorhersage der relativen Spaltgeschwindigkeiten verschiedener, beliebiger Oktapeptidsequenzen herangezogen werden.

### B) Vorhersage der relativen Proteolyserate von Oktamerpeptiden durch MMP-7

Aus den Inkrementdaten (siehe Fig. 6) wurden Einzelpeptidsubstrate mit unterschiedlicher erwarteter Halbwertszeit für die Proteolyse mittels MMP-7 abgeleitet. Diese Peptide wurden in Einzelassays mit MMP-7 unterschiedlich lange inkubiert und die Hydrolyserate mittels HPLC-ESI-MS analysiert, um die experimentellen Pseudo-Halbwertszeiten zu bestimmen. Es ergab sich eine gute Korrelation zwischen vorhergesagter und Pseudo-Halbwertszeit und experimentell bestimmten Wert (siehe Fig. 7). Dadurch ist es möglich, die relativen Spaltgeschwindigkeiten von Oktamerpeptiden bei MMP-7 Proteolyse anhand der Inkrementtabelle (siehe Fig. 6) vorherzusagen.

### C) Freisetzung eines niedermolekularen Substrats durch MMP-7 Proteolyse

MMP-7 spaltbare Peptidsubstrate wurden N-terminal kovalent an BSA (Rinderserumalbumin) gekoppelt und trugen C-terminal eine Biotinmarkierung. Das modifizierte BSA-Molekül wurde kovalent auf Luminex XMap™ beads (5 µm Durchmesser) gekoppelt, die unterschiedlich lange mit MMP-7 inkubiert wurden. Nach Ende der Inkubationszeit wurde die Biotinmenge pro bead durch Streptavidinfluoreszenz an die verbliebenen Biotinreste bestimmt. Die Streptavidinfluoreszenz pro bead wurde mit einem FACS=analogen System (Luminex 100 System) quantitativ bestimmt. Die Freisetzung des Biotins durch MMP-7 Aktivität konnte mit verschiedenen Peptidsubstraten gezeigt werden.

Dabei zeigte sich, dass die Abspaltung des C-terminalen, biotinylierten Peptidfragments nach unterschiedlich langer Inkubation mit MMP-7 zu einer zeitabhängigen Abnahme der Biotinylierung an der Beadoberfläche führte.

## Patentansprüche

1. Bioverbundmaterial mit lokaler, kontrollierter Freisetzung von Wirkstoffen, umfassend eine dreidimensionale Trägerstruktur auf Polymerbasis, sowie eine Matrixstruktur auf Polymerbasis, **dadurch gekennzeichnet, dass** die Wirkstoffe über die Matrixstruktur in das Bioverbundmaterial eingelagert sind und dass das Bioverbundmaterial durch Einbringen der Matrixstruktur in die Trägerstruktur erhältlich ist.

2. Bioverbundmaterial nach Anspruch 1, **dadurch gekennzeichnet, dass** die Trägerstruktur zumindest ein Polymer aufweist, das ausgewählt ist aus der Gruppe der synthetischen Polymere, insbesondere Polylactid, Polyglycolid, Polyethylenglycol, Polyvinylalkohol, Polymin, Polyurethan, polyHydroxybuttersäure, Polyethylenterephthalat, Polyterafluorethylen, Polypropolen, Gelatine, Kollagen, Fibrin, Stärke, Zellulose, Agarose, Alginat, Dextran, Chitosan, Hyaluronsäure, oder Mischungen davon.

3. Bioverbundmaterial nach einem der Ansprüche 1 oder 2, **dadurch gekennzeichnet, dass** die Matrixstruktur zumindest ein Polymer aufweist, das ausgewählt ist aus synthetischen Polymeren, insbesondere Polymin, Polyvinylalkohol, Polyethylenglycol, Polyurethan, Polyasparaginsäure, Polylysin; Biopolymeren, insbesondere Gelatine, Kollagenfragmente, Albumin, Dextran, Heparansulfat, Hyaluronsäure, Chitosan, Nucleinsäuren, Peptide, Lipide; oder biotechnologisch hergestellten polymeren Molekülen; oder Mischungen davon.

4. Bioverbundmaterial nach einem der Ansprüche 1 bis 3, **Dadurch gekennzeichnet, dass** sowohl die Trägerstruktur als auch die Matrixstruktur kollagenes Material oder Fragmente davon enthalten.

5. Bioverbundmaterial nach einem der Ansprüche 1 bis 4, **dadurch gekennzeichnet, dass** der Wirkstoff ausgewählt ist aus zumindest einer der Gruppen niedermolekulare Wirkstoffe, insbesondere Entzündungshemmer, Enzyminhibitoren, Antibiotika, Hormone; Wachstumsfaktoren, insbesondere Bone Morphogenetic Proteine, insbesondere BMP-2, -7; Chemokine; oder Mischungen davon.

6. Bioverbundmaterial nach einem der Ansprüche 1 bis 5, dadurch gekennzeichnest, dass der Wirkstoff durch eine unspezifische Bindung an die Matrixstruktur in das Biomaterial eingelagert ist.

7. Bioverbundmaterial nach einem der Ansprüche 1. bits 5, **dadurch gekennzeichnet, dass** der Wirkstoff über Peptide, die den Wirkstoff spezifisch oder unspezifisch binden, an die Matrixstruktur in das Biomaterial eingelagert ist.

8. Bioverbundmaterial nach Anspruch 7, **dadurch gekennzeichnet, dass** die den Wirkstoff bindenden Peptide über einen Linker an die Matrixstruktur, gebunden sind.

9. Bioverbundmaterial nach Anspruch 8, **dadurch gekennzeichnet, dass** die Anbindung des Linkers über Reaktionen der Reaktionspartner Thiol / Maleimid, N-Hydroxylamin / Aldehyd, Amin-Aldehyd erfolgt.

10. Bioverbundmaterial nach einem der Ansprüche 1 bis 5, **dadurch gekennzeichnet, dass** der Wirkstoff kovalent an die Matrixstruktur gebunden ist.

11. Bioverbundmaterial nach Anspruch 9, **dadurch gekennzeichnet**, das der Wirkstoff- über Peptide an die Matrixstruktur, gebunden ist, welche durch eine Matrixmetalloprotease (MMP), insbesondere MMP-7, MMP-1, MMP-3, MMP-8, MMP-9, MMP-10, MMP-13, spaltbar sind.

12. Bioverbundmaterial nach Anspruch 7, **dadurch gekennzeichnet, dass** der Wirkstoff über Peptide an die Matrixstruktur gebunden ist, welche die Wirkstoffe spezifisch binden.

13. Bioverbundmaterial nach Anspruch 12, **dadurch gekennzeichnet, dass** der Wirkstoff der Wachstumsfaktor BMP-2 (Bone Morphogenic Protein 2) ist, das über für BMP-2 spezifische Zyklopeptide spezifisch gebunden ist.

14. Bioverbundmaterial nach Anspruch 7, **dadurch gekennzeichnet dass** der Wirkstoff über ein an die Matrixstruktur kovalent gebundenes Peptid an die Matrixstruktur gebunden ist, wobei das Peptid durch eine Matrixmetalloprotease (MMP), insbesondere durch MMP-7, spaltbar ist.

15. Bioverbundmaterial nach einem der Ansprüche 7 bis 9 oder 12 und 13, **dadurch gekennzeichnet, dass** das Peptid, das den Wirkstoff bindet, von einem Protein abgeleitet ist, das ausgewählt ist aus bovinem Fetuin, bovinem BBP und TGFβR-2.

16. Bioverbundmaterial nach Anspruch 15, **dadurch gekennzeichnet, dass** das Peptid, das den Wirkstoff spezifisch bindet, eine Sequenz aufweiset, die ausgewählt ist aus einer der SEQ ID Nr. 1 bis 12 aus Fig. 4 und dem beigefügten Sequenzprotokoll.

17. Bioverbundmaterial nach Anspruch 14, **dadurch gekennzeichnet, dass** das kovalent an die Matrixstruktur gebundene Peptid eine Sequenz aufweist, die ausgewählt ist aus einer der SEQ ID Nr. 13 bis 20 aus Fig. 7 und dem beigefügten Sequenzprotokoll.

18. Verfahren zur Herstellung eines Bioverbundmaterials, umfassend eine dreidimensionale Trägerstruktur auf Polymerbasis, sowie eine Matrixstruktur auf Polymerbasis, mit den folgenden Schritten:
a) Bereitstellen von i) Trägermaterial auf Polymerbasis und ii) Matrixmaterial auf Polymerbasis;
b) Hydrophilisieren des Trägermaterials; und
c) Einbringen des Matrixmaterials in das hydrophilisierte Trägermaterial aus Schritt b) zur Herstellung eines Bioverbundmaterials mit einer Trägerstruktur und einer Matrixstruktur.

19. Verfahren nach Anspruch 18, **dadurch gekennzeichnet**, das in Schritt a) ein Matrixmaterial bereitgestellt wird, an das Wirkstoffe gebunden sind, zur Herstellung eines Bioverbundmaterials mit einer Trägerstruktur und einer wirkstoffdotierten Matrixstruktur.

20. Bioverbundmaterial nach einem der Ansprüche 1 bis 17, oder Bioverbundmaterials, das nach einen der Ansprüche 18 oder 19 hergestellt, ist, zur Verwendung zur Behandlung von degeneriertem oder erkranktem Gewebe.

21. Bioverbundmaterials nach einem der Ansprüche 1 bis 17, oder Bioverbundmaterial, das nach einem der Ansprüche 18 oder 19 hergestellt, ist, zur Verwendung für die lokale Abgabe von Chemotherapeutika.

22. Bioverbundmaterial nach Anspruch 21, zur Verwendung zur Behandlung von Krebs.

23. Verwendung eines Bioverbundmaterials nach einem der Anspruche 1 bis 17, oder eines Bioverbundmaterials, das nach einem der Ansprüche 18 oder 19 hergestellt ist, für die *in vitro* Zellkultur.

## Claims

1. Composite biomaterial with local, controlled release of active ingredients, comprising a three-dimensional polymer-based support structure, and a polymer-based matrix structure, **characterized in that** the active ingredients are incorporated into the composite biomaterial via the matrix structure and that the composite biomaterial is obtainable by introducing the matrix structure into the support structure.

2. Composite biomaterial according to Claim 1, **characterized in that** the support structure includes at least one polymer which is selected from the group of synthetic polymers, in particular polylactide, polyglycolide, polyethylene glycol, polyvinyl alcohol, polyimine, polyurethane, polyhydroxybutyric acid, polyethylene terephthalate, polytetrafluoroethylene, polypropylene, gelatine, collagen, fibrin, starch, cellulose, agarose, alginate, dextran, chitosan, hyaluronic acid, or mixtures thereof.

3. Composite biomaterial according to any of Claims 1 or 2, **characterized in that** the matrix structure includes at least one polymer which is selected from synthetic polymers, in particular polyimine, polyvinyl alcohol, polyethylene glycol, polyurethane, polyaspartic acid, polylysine; biopolymers, in particular gelatine, collagen fragments, albumin, dextran, heparan sulphate, hyaluronic acid, chitosan, nucleic acids, peptides, lipids; or biotechnologically prepared polymeric molecules; or mixtures thereof.

4. Composite biomaterial according to any of Claims 1 to 3, **characterized in that** both the support structure and the matrix structure comprise collagenous material or fragments thereof.

5. Composite biomaterial according to any of Claims 1 to 4, **characterized in that** the active ingredient is selected from at least one of the groups of low molecular weight active ingredients, in particular anti-inflammatory agents, enzyme inhibitors, antibiotics, hormones; growth factors, in particular bone morphogenetic proteins, in particular BMP-2, -7; chemokines; or mixtures thereof.

6. Composite biomaterial according to any of Claims 1 to 5, **characterized in that** the active ingredient is incorporated into the biomaterial by a nonspecific binding to the matrix structure.

7. Composite biomaterial according to any of Claims 1 to 5, **characterized in that** the active ingredient is incorporated into the biomaterial via peptides which bind the active ingredient specifically or nonspecifically to the matrix structure.

8. Composite biomaterial according to Claim 7, **characterized in that** the peptides binding the active ingredient are bound via a linker to the matrix structure.

9. Composite biomaterial according to Claim 8, **characterized in that** the attachment of the linker takes place by reactions of the reactants thiol/maleimide, N-hydroxylamine/aldehyde, amine-aldehyde.

10. Composite biomaterial according to any of Claims 1 to 5, **characterized in that** the active ingredient is covalently linked to the matrix structure.

11. Composite biomaterial according to Claim 9, **characterized in that** the active ingredient is linked to the matrix structure via peptides which are cleavable by a matrix metalloprotease (MMP), in particular MMP-7, MMP-1, MMP-3, MMP-8, MMP-9, MMP-10, MMP-13.

12. Composite biomaterial according to Claim 7, **characterized in that** the active ingredient is bound to the matrix structure via peptides which specifically bind the active ingredients.

13. Composite biomaterial according to Claim 12, **characterized in that** the active ingredient is the growth factor BMP 2 (bone morphogenic protein 2) which is specifically bound via cyclopeptides specific for BMP-2.

14. Composite biomaterial according to Claim 7, **characterized in that** the active ingredient is linked to the matrix structure via a peptide covalently linked to the matrix structure, where the peptide is cleavable by a matrix metalloprotease (MMP), in particular by MMP-7.

15. Composite biomaterial according to any of Claims 7 to 9 or 12 and 13, **characterized in that** the peptide which binds the active ingredient is derived from a protein which is selected from bovine fetuin, bovine BBP and TGFβR-2.

16. Composite biomaterial according to Claim 15, **characterized in that** the peptide which specifically binds the active ingredient has a sequence which is selected from one of the SEQ ID No. 1 to 12 from Fig. 4 and the appended sequence listing.

17. Composite biomaterial according to Claim 14, **characterized in that** the peptide covalently linked to the matrix structure has a sequence which is selected from one of the SEQ ID No. 13 to 20 from Fig. 7 and the appended sequence listing.

18. Process for producing a composite biomaterial comprising a three-dimensional polymer-based support structure, and a polymer-based matrix structure, with the following steps:
a) providing of i) polymer-based support material and ii) polymer-based matrix material;
b) hydrophilizing of the support material; and
c) introducing of the matrix material into the hydrophilized support material from step b) to produce a composite biomaterial with a support structure and a matrix structure.

19. Process according to either of Claim 18, **characterized in that** a matrix material to which active ingredients are bound is provided in step a) to produce a composite biomaterial with a support structure and an active ingredient-doped matrix structure.

20. Composite biomaterial according to any of Claims 1 to 17, or of a composite biomaterial which is produced according to any of Claims 18 or 19, for use in the treatment of degenerated or diseased tissue.

21. Composite biomaterial according to any of Claims 1 to 17, or of a composite biomaterial which is produced according to any of Claims 18 or 19, for use in the local delivery of chemotherapeutics.

22. Composite biomaterial according to claim 21, for use in the treatment of cancer.

23. Use of a composite biomaterial according to any of Claims 1 to 17, or of a composite biomaterial which is prepared according to any of Claims 18 or 19, for *in vitro* cell culturing.

## Revendications

1. Matériau biocomposite à libération locale et contrôlée de principes actifs, comprenant une structure tridimensionnelle formant support à base de polymères, ainsi qu'une structure formant matrice à base de polymères, **caractérisé en ce que** les principes actifs sont par l'intermédiaire de la structure formant matrice incorporés dans le matériau biocomposite, et que le matériau biocomposite peut être obtenu par introduction de la structure formant matrice dans la structure formant support.

2. Matériau biocomposite selon la revendication 1, **caractérisé en ce que** la structure formant support comprend au moins un polymère qui est choisi dans le groupe des polymères synthétiques, en particulier le polylactide, le polyglycolide, le polyéthylèneglycol, le poly(alcool vinylique), la polyimine, le polyuréthanne, le poly(acide hydroxybutyrique), le poly(téréphtalate d'éthylène), le polytétrafluoréthylène, le polypropylène, la gélatine, le collagène, la fibrine, l'amidon, la cellulose, l'agarose, l'alginate, le dextran, le chitosan, l'acide hyaluronique ou les mélanges de ceux-ci.

3. Matériau biocomposite selon l'une des revendications 1 ou 2, **caractérisé en ce que** la structure formant matrice comprend au moins un polymère qui est choisi parmi les polymères synthétiques, en particulier la polyimine, le poly(alcool vinylique), le polyéthylèneglycol, le polyuréthanne, le poly(acide asparagique), la polylysine ; les biopolymères, en particulier la gélatine, les fragments collagéniques, l'albumine, le dextran, le sulfate d'héparane, l'acide hyaluronique, le chitosan, les acides nucléiques, les peptides, les lipides ; ou les molécules polymères fabriquées par biotechnologie ; ou les mélanges de ceux-ci.

4. Matériau biocomposite selon l'une des revendications 1 à 3, **caractérisé en ce que** tant la structure formant support que la structure formant matrice contiennent un matériau collagénique ou des fragments de celui-ci.

5. Matériau biocomposite selon l'une des revendications 1 à 4, **caractérisé en ce que** le principe actif est choisi dans au moins l'un des groupes consistant en les principes actifs à faible masse moléculaire, en particulier les anti-inflammatoires, les inhibiteurs
enzymatiques, les antibiotiques, les hormones ; les facteurs de croissance, en particulier les protéines osseuses morphogénétiques, en particulier BMP-2, -7 ; les chimiokines ; ou les mélanges de ceux-ci.

6. Matériau biocomposite selon l'une des revendications 1 à 5, **caractérisé en ce que** le principe actif est incorporé dans le biomatériau par une liaison non-spécifique à la structure formant matrice.

7. Matériau biocomposite selon l'une des revendications 1 à 5, **caractérisé en ce que** le principe actif est incorporé dans le biomatériau par l'intermédiaire de peptides qui lient le principe actif d'une manière spécifique ou non-spécifique à la structure formant matrice.

8. Matériau biocomposite selon la revendication 7, **caractérisé en ce que** les peptides liant le principe actif sont liés à la structure formant matrice par l'intermédiaire d'un lieur.

9. Matériau biocomposite selon la revendication 8, **caractérisé en ce que** la liaison du lieur a lieu par l'intermédiaire de réactions des partenaires de réaction thiol / maléimide, N-hydroxylamine / aldéhyde, amine-aldéhyde.

10. Matériau biocomposite selon l'une des revendications 1 à 5, **caractérisé en ce que** le principe actif est lié par liaison covalente à la structure formant matrice.

11. Matériau biocomposite selon la revendication 9, **caractérisé en ce que** le principe actif est lié à la structure formant matrice par l'intermédiaire de peptides qui peuvent être clivés par une métalloprotéase matricielle (MMP), en particulier MMP-7, MMP-1, MMP-3, MMP-8, MMP-9, MMP-10, MMP-13.

12. Matériau biocomposite selon la revendication 7, **caractérisé en ce que** le principe actif est lié à la structure formant matrice par l'intermédiaire de peptides qui lient les principes actifs d'une manière spécifique.

13. Matériau biocomposite selon la revendication 12, **caractérisé en ce que** le principe actif est le facteur de croissance BMP-2 (protéine osseuse morphogénétique 2), qui est lié d'une manière spécifique par l'intermédiaire de cyclopeptides spécifiques du BMP-2.

14. Matériau biocomposite selon la revendication 7, **caractérisé en ce que** le principe actif est lié à la structure formant matrice par l'intermédiaire d'un peptide lié par liaison covalente à la structure formant matrice, le peptide pouvant être clivé par une métalloprotéase matricielle (MMP), en particulier par MMP-7.

15. Matériau biocomposite selon l'une des revendications 7 à 9 ou 12 et 13, **caractérisé en ce que** le peptide qui lie le principe actif dérive d'une protéine qui est choisie parmi la fétuine bovine, la BBP bovine et le TGFβR-2.

16. Matériau biocomposite selon la revendication 15, **caractérisé en ce que** le peptide qui lie le principe actif d'une manière spécifique présente une séquence qui est choisie parmi l'une des séquences SEQ ID NO:1 à 12 de la Fig. 4 et le protocole de séquence joint.

17. Matériau biocomposite selon la revendication 14, **caractérisé en ce que** le peptide lié par liaison covalente à la structure formant matrice présente une séquence qui est choisie parmi l'une des séquences SEQ ID NO:13 à 20 de la Fig. 7 et le protocole de séquence joint.

18. Procédé de fabrication d'un matériau biocomposite comprenant une structure tridimensionnelle formant support à base de polymères, ainsi qu'une structure formant matrice à base de polymères, comprenant les étapes suivantes :
a) mise à disposition i) d'un matériau formant support à base de polymères et ii) d'un matériau formant matrice à base de polymères;
b) hydrolyse du matériau formant support ; et
c) introduction du matériau formant matrice dans le matériau formant support hydrophilisé de l'étape b) pour fabriquer un matériau biocomposite comprenant une structure formant support et une structure formant matrice.

19. Procédé selon la revendication 18, **caractérisé en ce que**, dans l'étape a), on met à disposition un matériau formant matrice auquel sont liés des principes actifs, pour la fabrication d'un matériau biocomposite comprenant une structure formant support et une structure formant matrice dopées par les principes actifs.

20. Matériau biocomposite selon l'une des revendications 1 à 17, ou matériau biocomposite qui est fabriqué selon l'une des revendications 18 ou 19, pour utilisation dans le traitement de tissus dégénérés ou lésés.

21. Matériau biocomposite selon l'une des revendications 1 à 17, ou matériau biocomposite qui est fabriqué selon l'une des revendications 18 ou 19, pour utilisation pour l'administration locale d'agents chimiothérapeutiques.

22. Matériau biocomposite selon la revendication 21, pour une utilisation pour le traitement du cancer.

23. Utilisation d'un matériau biocomposite selon l'une des revendications 1 à 17 ou d'un matériau biocomposite qui est fabriqué selon l'une des revendications 18 ou 19 pour une culture cellulaire *in vitro.*
